# EUROPÄISCHE PATENTSCHRIFT

(11) **EP 4 512 448 B1**
(45) Veröffentlichungstag und Bekanntmachung des Hinweises auf die Patenterteilung: **24.06.2026**
(21) Anmeldenummer: 24194617.7
(22) Anmeldetag: 14.08.2024
(51) Int. Cl.: A61M 16/06

(54) **BEFESTIGUNGSVORRICHTUNG FÜR EIN PATIENTENINTERFACE, PATIENTENINTERFACE SOWIE SYSTEM ZUR BEATMUNG UND/ODER ATEMUNTERSTÜTZUNG**
FASTENING DEVICE FOR A PATIENT INTERFACE, PATIENT INTERFACE AND SYSTEM FOR VENTILATING AND/OR RESPIRATORY ASSISTANCE
DISPOSITIF DE FIXATION POUR INTERFACE PATIENT, INTERFACE PATIENT ET SYSTÈME DE VENTILATION ET/OU D'ASSISTANCE RESPIRATOIRE

(30) Priorität: 24.08.2023 DE 102023122679
(43) Veröffentlichungstag der Anmeldung: 26.02.2025
(73) Patentinhaber: Löwenstein Medical Technology S.A., 2557 Luxembourg (LU)
(72) Erfinder: Bechtel, Martin, 21423 Winsen / Luhe (DE); Gardein, Joachim, 38430 Icod de los Vinos (ES)
(74) Vertreter: Löwenstein Medical IP

(56) Entgegenhaltungen:
- WO-A1-2021/162631
- WO-A2-2023/043377
- US-A1- 2016 038 708
- US-A1- 2016 067 441
- US-A1- 2017 274 167
- US-A1- 2022 401 685
- US-B2- 9 878 118

## Beschreibung

Die vorliegende Offenbarung betrifft eine Befestigungsvorrichtung für ein Patienteninterface umfassend ein im Wesentlichen U-förmiges Stützelement mit einem Scheitelpunkt und einem ersten und zweiten Endabschnitt aus einem steifen, halbsteifen Material oder Mischungen hiervon, welches im bestimmungsgemäßen Gebrauch den Hinterkopf eines Patienten umrahmt.

Die vorliegende Offenbarung betrifft weiterhin ein Patienteninterface zur Verwendung mit einer offenbarungsgemäßen Befestigungsvorrichtung sowie ein System zur Beatmung und/oder Atemunterstützung umfassend ein Patienteninterface, einen Patientenschlauch sowie eine offenbarungsgemäße Befestigungsvorrichtung.

Unter Patienteninterfaces gemäß der vorliegenden Offenbarung werden in der Regel Atemmasken aus einem Maskenkörper und einem Maskenwulst verstanden. Diese können als Nasalmaske, als Full-Face-Maske, als Nasal-Pillow oder Nasal-Pong, als Mundstück oder als Hybrid-Maske umfassend ein Mundstück und ein Nasal-Pillow ausgebildet sein. Patienteninterfaces gemäß der vorliegenden Offenbarung werden hauptsächlich zur Behandlung schlafbezogener Atmungsstörungen, insbesondere dem obstruktiven Schlafapnoe-Syndrom, oder Beeinträchtigungen der Atempumpe, wie beispielsweise einer chronischobstruktiven Bronchitis (COPD), eingesetzt unter Verwendung kontinuierlichem positivem Atemwegsdruck (CPAP) oder nicht-invasiver Überdruckbeatmung. Das Patienteninterface ist dabei über einen Patientenschlauch mit einem Beatmungsgerät oder Atemtherapiegerät verbunden. Bevorzugt sind hierbei Patienteninterfaces, die klein in ihrem Durchmesser, niedrig in ihrer Höhe und gering in ihrem Gewicht sind. Diese haben den Vorteil, dass die durch den Patientenschlauch verursachten Hebelkräfte klein sind, das Totraumvolumen gering ist und das Patienteninterface bequem zu tragen ist.

Patienteninterfaces werden über Kopfbänderungen an dem Kopf eines Patienten befestigt. Diese sollen den Maskenkörper sicher am Kopf eines Patienten halten, wobei nur eine optimale Positionierung den erforderlichen Halt ermöglicht, ohne dass es zu Leckagen kommt. Oftmals werden hierzu Stirnstützen oder mehrere breite Gurte verwendet, welche zwar eine bessere Druckverteilung im Gesicht des Patienten ermöglichen, jedoch auch sehr präsent im Sichtfeld eines Patienten liegen und das Gesamtgewicht deutlich spürbar erhöhen. Durch vermehrte Anzahl an Auflageflächen im Gesicht des Patienten durch breite Gurte kann es weiterhin zu Hautreizungen, Hautallergien und Hauterosionen kommen, sowie zu Schürfstellen an empfindlicheren Stellen, wie den Ohren.

Die Nachfrage nach Befestigungsvorrichtungen für Patienteninterfaces, die auf Stirnstützen und breite Gurte oder Riemen verzichten, für den Patienten also angenehmer und komfortabler zu tragen sind und das Gesichtsfeld wenig einschränken, ist immens.

Die EP 3 085 405 offenbart eine Kopfmontur zur Befestigung einer Atemmaske mit einem ersten Riemen, der in bestimmungsgemäßem Gebrauch die Rückseite des Kopfs eines Patienten umfasst und eine im Wesentlichen kreisförmige Form annimmt, sowie einem Paar oberer und unterer Riemen, welche sich über bzw. unter dem Ohr eines Patienten erstrecken und eine Atemmaske aufnehmen können. Der erste Riemen umfasst hierbei eingekapselte Versteifungen sowie eine erste und zweite Gewebeschicht. Die gezeigten eingekapselten Versteifungen sind in der Lage sich längs ihrer Breite zu biegen, wobei eine Dehnung der gesamten Kopfmontur in einer Längsrichtung der Versteifung verhindert wird.

Die EP 2 373 368 B1 zeigt eine Kopfbänderung zur Verwendung mit einer Maske mit einem ersten Riemen, der bei Verwendung eine im Wesentlichen kreisförmige Form annimmt und die Rückseite des Kopfes eines Patienten aufnimmt, wobei ein Teil dieses ersten Riemens nicht dehnbar ist, sowie einem oberen und einem unteren Riemen, die sich oberhalb bzw. unterhalb des Ohrs eines Pateinten erstrecken, wobei der obere Riemen dehnbarer ist als der untere Riemen.

Weitere Kopfbänderungen des Stands der Technik sind den nachfolgenden Offenbarungen zu entnehmen, nämlich WO 2021/162631 A1, US 9 878 118 B2, US 2016/067441 A1, WO 2023/043377 A2, US 2022/401685 A1, US 2017/274167 A1 und US 2016/038708 A1.

Die gezeigten Möglichkeiten des Stands der Technik weisen die bereits erwähnten Nachteile von Stirnstützen bzw. mehrerer breiten Gurte im Gesicht des Patienten auf, wie ein eingeschränktes Sichtfeld des Patienten und insbesondere bei längerem Tragen das Hinterlassen von Druckstellen, Hautreizungen und Abschürfungen. Weiterhin sind die dort offenbarten Versteifungen in ihrer Dehnbarkeit eingeschränkt, was keine Anpassungen an unterschiedliche Kopfformen ermöglicht und beim Tragen, insbesondere in einer Liegeposition, unbequem ist. Ferner sind die gezeigten Versteifungen des Stands der Technik in Gewebeschichten eingekapselt, sodass deren Reinigung erschwert ist, was zu einer Ansammlung von Bakterien führen kann, was wiederum das Hautreizungs- und Allergiepotential fördert.

Der vorliegenden Erfindung lag daher die Aufgabe zugrunde, Befestigungsvorrichtungen für Patienteninterfaces bereitzustellen, die nicht mit den Nachteilen des Stands der Technik behaftet sind und dem Patienten ein angenehmes und komfortables Tragen der Patienteninterfaces, insbesondere auch in einer Liegeposition, ermöglichen. Dabei sollen die gezeigten Befestigungsvorrichtungen einen sicheren Halt der Patienteninterfaces bieten, sowie eine optimale Positionierung, ohne dass das Sichtfeld des Patienten zu stark eingeschränkt wird und ohne, dass es zu Hautreizungen, Hautallergien, Hauterosionen und Schürfstellen kommt.

Die Erfindung ist in den angehängten Ansprüchen offenbart.

Demgemäß wurde eine Befestigungsvorrichtung für ein Patienteninterface gefunden, umfassend ein im Wesentlichen U-förmiges Stützelement mit einem Scheitelpunkt, sowie einem ersten und einem zweiten Endabschnitt, ausgebildet und eingerichtet im bestimmungsgemäßen Gebrauch den Hinterkopf eines Patienten zu umrahmen, wobei das U-förmige Stützelement ein steifes, ein halbsteifes Material oder Mischungen hiervon umfasst.

Das offenbarungsgemäße U-förmige Stützelement ist dabei so ausgebildet, dass es kein Textilgewebe umfasst, demnach ohne großen Aufwand keimfrei zu reinigen ist und weniger Verschleiß aufgrund von Abnutzungen aufweist. Weiterhin ist das verwendete Material steif genug, um auch während des Nicht-Gebrauchens durch einen Patienten seine Form beizubehalten. Dies ermöglicht dem Patienten eine mühelosere Handhabung, indem die Befestigungsvorrichtung einfacherer aufzusetzen bzw. anzulegen ist. Dies ist bei Systemen, die mehrere Textilgurte oder Textilriemen umfassen, oftmals schwierig, da sich diese Systeme verknäulen und aufwendig vor dem Nutzen entwirrt werden müssen.

Unter U-förmig gemäß der vorliegenden Offenbarung kann sowohl ein bogenförmiges U-förmiges Stützelement verstanden werden, bei welchem der untere Teil im Abschnitt des Scheitelpunkts als Halbkreis ausgebildet ist. Dabei sind solche U-förmigen Stützelemente bevorzugt, bei denen solch ein Halbkreis einen Radius zwischen 4 cm und 8 cm aufweist, insbesondere einen Radius zwischen 5 und 7 cm. Bevorzugt sind jedoch solche U-förmigen Elemente, bei den die U-Form keinen Bogen oder Halbkreis ausbildet, sondern im Abschnitt des Scheitelpunkts ein Plateau aufweist, die Form also in Richtung eines Rechtecks mit abgerundeten Kanten abweicht. Solch geformten Elemente haben den Vorteil, dass sie sich noch besser an die Kopfform eines Patienten anschmiegen.

Trotz der Verwendung von steifem oder halbsteifem Material weist das im Wesentlichen U-förmige Stützelement eine Torsion gegenüber einer Geraden auf, welche durch den Scheitelpunkt und mittig zu dem ersten und zweiten Endabschnitt verläuft. Hierdurch wird eine gute Anpassung an die jeweilige Lage eines Patienten ermöglicht, wie beispielsweise dem Liegen auf dem Rücken oder dem Liegen auf der Seite.

Weiterhin ist es ebenfalls vorteilhaft, dass das offenbarungsgemäße U-förmige Stützelement eine Dehnbarkeit gegenüber einer Geraden aufweist, welche durch den Scheitelpunkt und mittig zu dem ersten und zweiten Endabschnitt verläuft. Dies erlaubt eine gute Anpassung an die jeweilige Kopfform eines Patienten, insbesondere im Hinblick auf dessen Kopfumfang, ohne dabei Druckstellen zu hinterlassen. Die offenbarungsgemäße Befestigungsvorrichtung ist dadurch grundsätzlich bei vielen Patienten einsetzbar, ohne dass es spezieller verschiedener Größen des U-förmigen Stützelements bedarf.

In einer besonders bevorzugten Ausführungsform der offenbarungsgemäßen

Befestigungsvorrichtung ist der Scheitelpunkt des U-förmigen Stützelements eingerichtet, bei einer Anwendung am Patienten mittig an dessen Hinterhauptbein anzuliegen. Der erste und der zweite Endabschnitt des U-förmigen Stützelements sind alternativ oder zusätzlich eingerichtet, bei einer Anwendung am Patienten jeweils flach seitlich an dessen Kopf über den Ohren positioniert zu sein. Bei dieser Ausgestaltung der Befestigungsvorrichtung liegen die direkten Berührungspunkte des U-Förmigen Stützelements in bestimmungsgemäßem Gebrauch also an flachen Stellen des Kopfes an, wie dem Hinterkopf und seitlich über den Ohren. Dies führt dazu, dass es zu keinerlei Hautreizungen oder wunden Stellen durch Reibung oder Druck kommt.

Besonders gut geeignet sind U-förmige Stützelemente, bei denen der Abstand zwischen dem ersten und dem zweiten Endabschnitt im Zustand des Nicht-Gebrauchens 8 cm bis 15 cm beträgt, insbesondere 10 bis 13 cm. Weiterhin sind solche U-förmigen Stützelemente vorteilhaft, bei denen der Abstand zwischen Scheitelpunkt und erstem bzw. zweiten Endabschnitt im Zustand des Nicht-Gebrauchens 14 bis 21 cm beträgt, insbesondere 16 bis 19 cm.

In bestimmten Ausführungsformen, insbesondere wenn das verwendete Patienteninterface als Full-Face-Maske ausgebildet ist, kann es alternativ bevorzugt sein, wenn das U-förmige Stützelement in bestimmten Vorab-Größen, beispielsweise klein (small), mittel (medium) und groß (large) ausgebildet ist, um eine noch bessere Anpassung an verschiedene Kopfgrößen zu ermöglichen.

Weiterhin haben sich solche Befestigungsvorrichtungen als besonders geeignet erwiesen, die ferner mindestens ein Übergangselement umfassen, welches das U-förmige Stützelement mit dem Patienteninterface verbindet. Dieses mindestens eine Übergangselement ist dabei bevorzugt ebenfalls aus einem steifen oder halbsteifen Material oder einer Mischung hiervon ausgebildet. Besonders gut geeignet sind Befestigungsvorrichtungen, die zwei Übergangselemente umfassen, die jeweils linksseitig und rechtsseitig des Patienteninterfaces verlaufen. Die Übergangselemente aus steifem oder halbsteifem Material bieten der Befestigungsvorrichtung die benötigte Steifheit bzw. Starrheit, um auch ohne Stirnstütze oder breite Gurte den erforderlichen Halt und die optimale Positionierung zu bieten und den Hebelkräften beim Anschluss eines Patientenschlauchs entgegenzuwirken.

Um das Sichtfeld eines Patienten möglichst wenig einzuschränken, haben sich solche Befestigungsvorrichtungen als besonders geeignet erwiesen, die ferner mindestens einen Stoffstrang umfassen, welcher der Verbindung des U-förmigen Stützelements mit dem mindestens einen Übergangselement dient. In einer alternativen bevorzugten Ausgestaltung umfasst die Befestigungsvorrichtung zwei Stoffstränge. Bevorzugt ist hierbei wiederum, wenn diese zwei Stoffstränge in Ausführungsformen mit zwei Übergangselementen verwendet werden.

Die Stoffstränge sind flexibel und weich ausgebildet und verlaufen in der offenbarungsgemäßen Befestigungsvorrichtung im Gesicht des Patienten, und zwar an den Stellen, an denen eine übermäßige Starrheit der Struktur nicht benötigt wird und sogar als unangenehm empfunden wird, wie dem empfindlichen Wangenbereich und in Augennähe. Diese flexiblen weichen Stoffstränge erhöhen den Tragekomfort für den Patienten enorm und schränken das Sichtfeld des Patienten nicht ein. Weiterhin kann durch die Verwendung von leichten Stoffsträngen das Gesamtgewicht der Befestigungsvorrichtung reduziert werden, was ebenfalls zu einem angenehmeren Tragegefühl beiträgt. Die Flexibilität der Stoffstränge ermöglicht weiterhin eine bessere Anpassung an die jeweilige Kopfform des Patienten, sodass ein fester optimaler Sitz des Patienteninterfaces garantiert werden kann.

Zusätzlich können der eine oder die zwei Stoffstränge der offenbarungsgemäßen Befestigungsvorrichtung Polsterelemente umfassen, wobei diese vorzugsweise an der patientenzugewandten Innenseite lösbar angebracht sind.

Zur Verbindung des U-förmigen Stützelements mit dem mindestens einen Übergangselement durch einen ersten oder alternativ durch einen ersten und einen zweiten Stoffstrang, hat es sich als vorteilhaft erwiesen, wenn das U-förmige Stützelement ein Aufnahmeelement am Scheitelpunkt und Aufnahmeelemente an den beiden Endabschnitten aufweist.

Weiterhin ist es hierbei von Vorteil, wenn alternativ oder zusätzlich auch das mindesten eine Übergangselement oder alternativ das erste und das zweite Übergangselement mindestens ein Aufnahmeelement umfassen. Besonders gut geeignet sind Übergangselemente, die ein erstes und ein zweites Aufnahmeelement zur Aufnahme des Stoffstrangs oder der Stoffstränge umfassen.

In bevorzugten Ausgestaltungen sind die Aufnahmeelemente schlitzförmig ausgebildet, wobei die Schlitze eine Länge zwischen 0,5 mm und 6 mm, insbesondere zwischen 2 mm und 4 mm, aufweisen und eine Breite zwischen 10 mm und 19 mm, insbesondere zwischen 13 und 16 mm.

Die Verbindung des U-förmigen Stützelements und des mindestens einen Übergangselements über lösbare Stoffstränge bietet den Vorteil, dass diese einfach voneinander getrennt werden können, was die Reinigung der steifen oder halbsteifen Elemente und auch der Stoffstränge erleichtert. Die Komponenten können so separat gereinigt und desinfiziert werden, wobei halbsteife oder steife Materialien gegenüber Stoffsträngen andere Reinigungsmittel sowie Konzentrationen bevorzugen, ohne dass es zu Materialabnutzung oder Materialbeschädigungen kommt. Weiterhin erlaubt die lösbare Verbindung auch ein Auswechseln oder Ersetzen einzelner Komponenten, sollte es zum Verschleiß dieser kommen.

Besonders geeignet sind Ausgestaltungen der offenbarungsgemäßen Befestigungsvorrichtung, bei denen die Aufnahmeelemente durch Einschub- oder Durchführelemente gebildet werden. Dies können beispielsweise Materialaussparungen in dem U-förmigen Stützelement und den Übergangselementen sein oder auch Ausstülpungen zum Einhaken, die aus dem steifen oder halbsteifen Material selbst gebildet sind. Dies bietet den Vorteil einer einfachen Befestigung, bei der auf Ösen, Druckknöpfe, Haken oder ähnliches verzichtet werden kann, welche beim Tragen der Befestigungsvorrichtung aufgrund möglicher Reibung unangenehm für den Patienten sein könnten. Die Durchführung der Stoffstränge bei der Variante mit Materialaussparungen erfolgt dabei besonders bevorzugt in der Art, dass auf der patientenzugewandten Seite keine Erhöhung entsteht, bei der der Stoffstrang über dem U-förmigen Stützelement liegt, der Übergang also möglichst flach bleibt, sodass es auch hier nicht zu Druckstellen oder Reibung kommt.

Die Durchführelemente im U-förmigen Stützelement sind in geeigneten Ausgestaltungen der offenbarungsgemäßen Befestigungsvorrichtung zweiteilig ausgebildet, insbesondere als zwei schlitzförmige Materialaussparungen. Es hat sich hierbei als vorteilhaft erwiesen, wenn die beiden Schlitzförmigen Aussparungen parallel zueinander verlaufen.

Alternativ oder zusätzlich sind Befestigungsvorrichtungen besonders gut geeignet, bei denen das mindestens eine Übergangselement, insbesondere das erste und das zweite Übergangselement, aus einem Material ausgebildet sind, das eine höhere Steifigkeit als das U-förmige Stützelement aufweist.

Die Übergangselemente der offenbarungsgemäßen Befestigungsvorrichtung sitzen nahe am, insbesondere am, Patienteninterface, wo nach Anschluss eines Patientenschlauchs die Hebelkräfte stärker wirken, sodass hier eine gewisse Steifigkeit des Systems notwendig ist.

Sowohl im Falle des U-förmige Stützelements als auch des mindestens einen, insbesondere des ersten und zweiten, Übergangselements umfasst das steife oder halbsteife Material Polypropylen, Polyethylen, Polymethylpenten, Polycarbonat, Polystyrol, Polyisobutylen, Acrylnitril-Butadien-Styrol (ABS), Polyvinylidenfluorid, Polycaprolacton, Polyethylenterephthalat, Polybuthylenterephthalat, Ethylen-Chlortrifluorethylen, Polybenzimidazol, Polyoxymethylen, Ethylen-Vinylacetat-Copolymer, Styrol-Acrylnitril-Copolymer, Kohlenstoffnanoröhrchen, Celluloseacetatbutyrat, thermoplastische Elastomere und/oder Mischungen hiervon.

Die genannten Materialien sind im Vergleich zu Textil besser und einfacher zu reinigen und weisen eine höhere Robustheit auf, insbesondere in Bezug auf Abrieb.

Das verwendete steife und/oder halbsteife Material des offenbarungsgemäßen U-förmigen Stützelements und auch des mindestens einen, insbesondere des ersten und zweiten, Übergangselements ist bevorzugt in einer Dicke von 0,5 mm bis 4 mm, insbesondere 1,5 mm bis 2,5 mm, ausgebildet. Diese Materialdicken erlauben, dass die Materialien robust genug sind, um den Hebelkräften eines Patientenschlauchs standhalten können, aber auch flexibel genug, dass die offenbarungsgemäße Befestigungsvorrichtung bequem und komfortabel für den Patienten ist.

In geeigneten Ausgestaltungen der offenbarungsgemäßen Befestigungsvorrichtung umfasst das mindestens eine, insbesondere das erste und zweite Übergangselement, ferner jeweils ein Anschlusselement zur Verbindung bzw. zur Aufnahme eines Patienteninterfaces. Das Anschlusselement kann dabei in Form von Haken, eines Eindrehmechanismus, eines Klettsystems oder eines Einrastmechanismus (Klicksystems) ausgebildet sein. Besonders bevorzugt ist hierbei ein Einrastmechanismus. Dieser ist bei Bedarf möglichst einfach und schnell zu lösen, beispielsweise wenn der Patient Panik bekommt, etwas trinken möchte oder ähnliches.

In bevorzugten Ausgestaltungen der offenbarungsgemäßen Befestigungsvorrichtung umfassen die Übergangselemente an der patientenabgewandten Seite Griffelemente, sodass der Patient die Übergangselemente besser greifen kann, um sie mit dem Patienteninterface zu verbinden. Bevorzugt sind diese Griffelemente als Erhöhungen ausgebildet. Alternativ oder zusätzlich umfassen die Griffelemente eine strukturierte Oberfläche, die beispielsweise lamellenartig oder angeraut ausgebildet ist, sodass der Patient diese mit seinen Fingern gut umfassen kann.

Um die Übergangselemente möglichst materialsparend gestalten zu können, haben sich im Wesentlichen L-förmige Übergangselemente als besonders gut geeignet erwiesen. Weiterhin bietet diese Form eine möglichst gute Anpassung an die Knochenstruktur des Unterkiefers eines Patienten und wird daher vom Patienten als angenehm zu tragen empfunden.

In bevorzugten Ausgestaltungen weisen die Seitenlängen der L-förmigen Übergangselemente ein Verhältnis von 1,5 : 1 auf, wobei die Aufnahmeelemente bevorzugt an den Endabschnitten der kürzeren Seite liegen. Die Aufnahmeelemente sind in bevorzugten Ausführungsformen als einteilige schlitzförmige Materialaussparungen ausgebildet. Diese können hierbei parallel verlaufen oder auch um 90° versetzt. Für einen optimalen Verlauf des ersten und zweiten Stoffstrangs sind bei Nasalmasken parallel angeordnete Aufnahmeelemente bevorzugt, bei Full-Face-Masken haben sich Aufnahmeelemente als besonders gut geeignet erwiesen, die im Wesentlichen um etwa 90° versetzt angeordnet sind.

In geeigneten Ausführungsformen des L-förmigen Übergangselements spannen die Schenkel in Form der längeren und kürzeren Seite eine Fläche xy auf, wobei die L-förmigen Übergangselemente bevorzugt nicht flach in dieser Ebene ausgebildet sind. Vorteilhaft sind L-förmige Übergangselemente, bei denen einer der Schenkel eine Erhebung in die z-Ebene ausbildet. Die Gesamtstruktur der L-förmigen Übergangselemente kann sich durch die sich so ergebende geschwungene Form besonders gut an die Knochenstruktur des Unterkiefers eines Patienten anschmiegen.

In bevorzugten Ausgestaltungen sind die Übergangselemente mit mindestens einem Textilbezug versehen. Alternativ und oder zusätzlich können die Übergangselemente Polsterelemente umfassen. Die Übergangselemente können mit dem mindestens einen Textilbezug umwickelt sein, wobei dieser über Nähte, Klettverschlüsse, Reisverschlüsse, Druckknöpfe oder Ösen an dem mindesten einen Übergangselement befestig ist. Besonders bevorzugt sind Ausgestaltungen, bei denen eine schlauchartige Textilröhre über ein Übergangselement gestülpt wird und es keiner Verschlussverbindung bedarf. Diese Verschlussverbindungen neigen dazu Erhöhungen zu bilden, was der Patient beim Tragen als unangenehm empfindet und was ebenfalls zu Hautreizungen und Druckstellen führen kann. In jedem Fall sind Ausführungsformen bevorzugt, bei denen der Textilbezug ohne Klebstoffe an den Übergangselementen befestigt wird. Klebstoffe setzen mit der Zeit häufig Lösemittel frei oder zersetzen sich, was zu Hautallergien führen kann, insbesondere bei Patienten mit sensibler Haut.

Der offenbarungsgemäße Stoffstrang, insbesondere der erste und zweite Stoffstrang, sind in einer geeigneten Ausgestaltung mindestens dreilagig aufgebaut mit einer patientenzugewandten Innenlage, mindestens einer Zwischenlage und einer Außenlage, welche vom Patienten abgewandt liegt. In einer bevorzugten Ausgestaltung umfassen die Stoffstränge ein laminiertes Verbundmaterial, wobei die mindestens drei Lagen mittels Flammkaschierung miteinander verbunden sind. Solch ein Verfahren bietet den Vorteil, dass die Lagen fest miteinander verbunden sind, ohne dass Kleb- oder Leimstoffe verwendet werden müssen, welche auch in diesem Fall zu Hautallergien oder Hautreizungen führen könnte.

Alternativ oder zusätzlich sind solche Stoffstränge bevorzugt, welche abgerundete Kanten aufweisen, was den Tragekomfort für den Patienten erhöht und insbesondere an empfindlichen Stellen, wie den Ohren, nicht zu Schürfstellen führt.

Für einen besonders angenehmen Tragekomfort sind Stoffe bevorzugt, die eine glatte Oberflächenstruktur aufweisen und atmungsaktiv sind. Idealerweise sind diese Materialien weiterhin leicht zu reinigen. Besonders geeignet sind hierfür Stoffstränge, deren Innen- oder Außenlage, oder Innen- und Außenlage, ein synthetisches Textilgewebe oder cellulosische Chemiefasern aufweisen. Diese können Polyamide, regenerierte Polyamide, Bio-Polyamide, Polyethylenterephthalat, regeneriertes Polyethylenterephthalat, Polyurethan, Polypropylen, Acrylnitril, Viskose, Lyocell, Rayon, Modal oder Mischungen hiervon umfassen.

Die in den Stoffsträngen eingesetzten Textilien können hierbei gefärbt sein, was neben dem ansprechenderen optischen Eindruck dem Patienten auch eine Hilfestellung sein kann, wenn beispielsweise unterschiedliche Stoffstränge für verschiedene Tageszeiten in Kombination mit unterschiedlichen Patienteninterfaces verwendet werden (tagsüber, nachts beim Schlafen), wobei die unterschiedlich gefärbten Textilstränge hierbei möglicherweise auch anders in ihrer Breite oder Zugfestigkeit ausgebildet sein können. Alternativ oder zusätzlich können die unterschiedlich eingefärbten Stoffstränge auch andere Auflageflächen auf der Haut eines Patienten aufweisen, sodass durch einen Wechsel der Stoffstränge die jeweiligen Hautstellen entlastet werden können.

Dabei kann in der Herstellung solcher Stoffstränge das bereits gefertigte Textil eingefärbt werden oder alternativ das für die Herstellung verwendete Garn. In bevorzugten Ausgestaltungen der offenbarungsgemäßen Befestigungsvorrichtung sind die synthetischen Textilgewebe der Stoffstränge mittels Spinnfärbung (dope dyed oder solution dyeing) eingefärbt. Dabei werden Farbpigmente der Kunststoffschmelze schon bei Herstellung der Filamente zugefügt, wobei das Verfahren dem Fachmann geläufig ist. Die Spinnfärbung hat sich für die offenbarungsgemäßen Befestigungsvorrichtungen als besonders vorteilhaft erwiesen, um die Fasern der Textilstränge komplett durchzufärben und nicht nur eine Anhaftung der Farbpigmente auf der Textiloberfläche zu erhalten. Die verwendeten Stoffstränge sind häufigen Reinigungsprozessen ausgesetzt, um die Befestigungsvorrichtung keimfrei halten zu können. Demnach kann mit dem gezeigten Verfahren eine Farbechtheit auch nach mehreren Waschzyklen aufrechterhalten werden. Grundsätzlich weisen durch Spinnfärbung gefärbte Textilien im Vergleich zu nachträglich eingefärbten eine deutlich höhere Lichtechtheit und Langlebigkeit der Farbpigmente auf. Die so gefärbten Stoffstränge bieten dem Patienten weiter den Vorteil, dass es zu weniger Hautreizungen und Allergien kommt. Bei nachträglich gefärbten Textilien, bei denen die Farbpigmente nur auf der Oberfläche haften, ist die Wahrscheinlichkeit, dass sich diese durch Schweiß, Reibung und Wärme von der Faser lösen, deutlich erhöht. Weiterhin zeigen die durch Spinnfärbung gefärbten Stoffstränge der offenbarungsgemäßen Befestigungsvorrichtung eine geringere Umweltbelastung und bessere Biokompatibilität auf.

In geeigneten Ausgestaltungen der offenbarungsgemäßen Befestigungsvorrichtung umfassen der erste, insbesondere der erste und der zweite, Stoffstrang ferner eine Lasche, um in das Aufnahmeelement eingeführt werden zu können. Die Lasche kann hierbei mit dem jeweiligen Stoffstrang über ein Verbindungselement verbunden sein. Hierfür sind alle dem Fachmann geläufigen Verbindungselemente wie Haken, Druckknöpfe, Knöpfe, Einrastmechanismen, Magneten, Verschweißen und ähnliches denkbar. Besonders bevorzugt sind Ausgestaltungen, bei denen die Lasche mittig an die längere Seite des jeweiligen Stoffstrangs genäht ist, insbesondere so, dass die Lasche und der Stoffstrang lediglich aneinandergrenzen und nicht überlappen, sodass sich keine Erhöhung auf dem Stoffstrang bildet, welche vom Patienten beim Tragen als unangenehm empfunden wird und zu Druckstellen führt.

Alternativ sind Ausgestaltungen der offenbarungsgemäßen

Befestigungsvorrichtung geeignet, bei der die Lasche so mit dem Stoffstrang verbunden ist, dass diese nicht direkt aneinandergrenzen, sondern die Lasche relativ zum Stoffstrang bewegbar ist, insbesondere dass eine Größenanpassung an verschiedene Kopfgrößen möglich ist. Solch ein Verbindungselement kann beispielsweise durch einen Gummizug, eine Kordel mit Feststellmechanismus oder besondere Ausgestaltungen der oben genannten Verbindungselemente erfolgen.

Alternativ oder zusätzlich kann vorgesehen sein, dass die Laschen, insbesondere die patientenzugewandte Seite der Lasche, ein lösbares Polsterelement umfasst. Dies ist besonders in den Varianten der bewegbaren Laschen bevorzugt, wenn diese im Nacken eines Patienten aufliegen.

Weiterhin sind Ausgestaltungen bevorzugt, bei denen die Stoffstränge, insbesondere der Stoffstrang, der am Oberkopf eines Patienten verläuft, ein Kennzeichenelement aufweist, welches mittig zu dem ersten und zweiten Ende des Stoffstrangs verläuft. Dieses Kennzeichenelement liegt in bestimmungsgemäßem Gebrauch, das heißt wenn der Stoffstrang durch die Aufnahmeelemente des U-förmigen Stützelements geführt ist, mittig zu den Endabschnitten des U-förmigen Stützelements und bietet dem Patienten eine Hilfestellung die Befestigungsvorrichtung so auf dem Kopf zu platzieren, dass das Kennzeichenelement mittig am Hinterkopf liegt. Besonders vorteilhaft ist es hierbei, wenn das Kennzeichenelement ebenfalls zu keiner Erhöhung auf dem Stoffstrang führt, die zu Druckstellen führen kann. Gleichzeitig soll das Kennzeichenelement jedoch nicht nur über den visuellen Sinn wahrgenommen werden können, sondern auch durch den Tastsinn, um auch blinden Patienten als Hilfsmittel zu dienen. In bevorzugten Ausgestaltungen ist das Kennzeichenelement eine flache Naht, die sich farblich vom Stoffstrang abheben kann, aber auch mit den Fingern spürbar ist. Alternativ sind andere Kennzeichenelemente denkbar, die zu einer an diesem Punkt wahrnehmbaren veränderten Oberfläche führen, wie beispielsweise Vertiefungen, angeraute Stellen an der dem Patienten abgewandten Seite oder Ähnliches.

Besonders gut geeignet sind offenbarungsgemäße Befestigungsvorrichtungen, bei denen der erste, insbesondere der erste und der zweite Stoffstrang, an ihrem ersten und zweiten Strangende ein Fixierungselement umfassen. Diese Fixierungselemente sollen die Übergangselemente lösbar mit den Stoffstränge verbinden. Auch hier sind alle dem Fachmann geläufigen Fixierungselemente denkbar, wie beispielsweise Haken, Magnete, Druckknöpfe, Einrastelemente und dergleichen. In einer bevorzugten Ausgestaltung haben sich solche Fixierungselemente als besonders geeignet erwiesen, die es erlauben, neben ihrer Funktion der Fixierung, gleichzeitig eine optimale Größenanpassung an die Kopfform des Patienten zu ermöglichen. In einer bevorzugten Ausgestaltung umfassen das erste und das zweite Strangende der Stoffstränge daher Klettvorrichtungen, die derart ausgebildet sind, dass Hakenbänder an den Strangenden des Stoffstrangs angebracht sind, insbesondere an die Strangenden genäht sind. Bevorzugt ist die Textiloberfläche der gesamten Stoffstränge derart ausgebildet, dass sie als Gegenpart zum Hakenband wirken kann, sodass dieses an jeder beliebigen Stelle des Stoffstrangs fixiert werden kann. Dadurch ist die Größenanpassung an die jeweilige Kopfform des Patienten durch einfaches Versetzen des Strangendes, welches das Hakenband aufweist, möglich.

Die der Erfindung zugrunde liegende Aufgabe wird weiterhin gelöst durch ein Patienteninterface zur Verwendung mit einer erfindungsgemäßen Befestigungsvorrichtung.

In bevorzugten Ausgestaltungen umfasst das offenbarungsgemäße

Patienteninterface mindestens ein Eingliederungselement, insbesondere ein erstes und ein zweites Eingliederungselement. Dieses dienen dazu, die Anschlusselemente des ersten oder des zweiten Übergangselements, bevorzugt des ersten und des zweiten Übergangselements, aufzunehmen. Bevorzugt sind die Eingliederungselemente als Klicksystem über einen Einrastmechanismus ausgebildet. Alternativ sind auch Haken, Eindrehmechanismen, Klettsysteme oder ähnliche dem Fachmann geläufige Verbindungssysteme möglich. Der bevorzugte Einrastmechanismus über ein Klicksystem bietet den Vorteil, dass dieser bei Bedarf möglichst einfach und schnell zu lösen ist, beispielsweise wenn der Patient Panik bekommt, etwas trinken möchte oder ähnliches.

In geeigneten Ausgestaltungen des offenbarungsgemäßen Patienteninterfaces umfasst dieses ein Anbindungselement, um einen Patientenschlauch aufnehmen zu können. Bevorzugt sind hierbei Anbindungselemente, die über ein Kugelgelenk mit den offenbarungsgemäßen Patienteninterfaces verbunden sind. In solchen Ausführungsformen umfasst das Patienteninterface demnach eine Vertiefung bzw. Materialaussparung, die zur Aufnahme des Kugelgelenks geeignet ist. Die der Erfindung zugrunde liegende Aufgabe wird ferner gelöst durch ein System zur Beatmung und/oder Atemunterstützung umfassend ein offenbarungsgemäßes Patienteninterface, einen Patientenschlauch und eine erfindungsgemäße Befestigungsvorrichtung, wobei das offenbarungsgemäße Patienteninterface und ein Beatmungsgerät und/oder das Atemtherapiegerät über den Patientenschlauch gasleitend miteinander in Verbindung stehen.

Weitere Merkmale und Vorteile der Offenbarung ergeben sich aus der nachfolgenden Beschreibung, in der Ausführungsbeispiele anhand von schematischen Zeichnungen beispielhaft erläutert werden, ohne dadurch die Erfindung zu beschränken.

### Dabei zeigen

Figur 1 einen schematischen Aufbau einer Ausführungsvariante eines offenbarungsgemäßen U-förmigen Stützelements;
Figur 2 einen schematischen Aufbau einer Ausführungsform eines offenbarungsgemäßen Übergangselements;
Figur 3 einen schematischen Aufbau einer Ausführungsform eines offenbarungsgemäßen Stoffstrangs;
Figur 4 einen schematischen Aufbau einer Ausführungsform einer offenbarungsgemäßen Befestigungsvorrichtung und
Figur 5 einen schematischen Aufbau einer Ausführungsform offenbarungsgemäßer Übergangselemente verbunden mit einem offenbarungsgemäßen Patienteninterface.

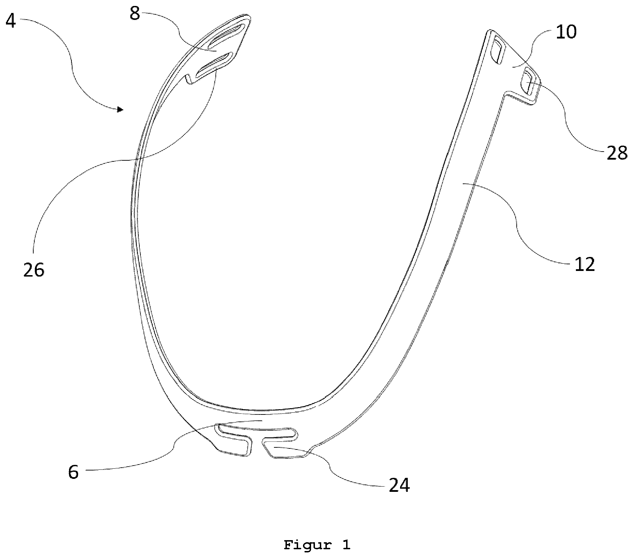

Figur 1 zeigt ein offenbarungsgemäßes U-förmiges Stützelement 4 mit einem Scheitelpunkt 6 sowie einem ersten und einem zweiten Endabschnitt 8 und 10. In der hier gezeigten Ausführungsform weist das U-förmige Stützelement 4 im Bereich des Scheitelpunkts 6 ein Plateau auf. Das U-förmige Stützelement 4 besteht in der gezeigten Variante aus einem steifen Material 12, welches die U-Form auch bei Nicht-Gebrauchen durch einen Patienten beibehält. In der gezeigten Ausführungsform ist eine Torsion des U-förmige Stützelements 4 gegenüber einer Geraden möglich, welche durch den Scheitelpunkt 6 und mittig zu dem ersten und zweiten Endabschnitt 8 und 10 verläuft, die beiden Arme des U-förmigen Stützelements 4 sind also zueinander verdrehbar. Weiterhin ist das U-förmige Stützelement 4 dehnbar gegenüber einer Geraden, welche durch den Scheitelpunkt 6 und mittig zu dem ersten und zweiten Endabschnitt 8 und 10 verläuft, die Arme des U-förmigen Stützelements 4 sind also aufeinander zu und voneinander weg bewegbar. In der gezeigten Ausführungsform des U-förmigen Stützelements 4 liegt der Scheitelpunkt 6 bei Anwendung am Patienten mittig am Hinterhauptbein an und die beiden Endabschnitte 8 und 10 seitlich am Kopf über den Ohren positioniert. In der gezeigten Variante des erfindungsgemäßen U-förmigen Stützelements 4 umfasst dieses im Bereich des Scheitelpunkts 6 ein Aufnahmeelement 24, welches hier in Form eines Einschubelements ausgebildet ist, bei dem das steife Material 12 zwei Ausstülpungen bildet, in die eine Lasche 52 eines Stoffstrangs 20 (hier nicht gezeigt) eingefädelt werden kann, sodass eine lösbare Verbindung zwischen dem erfindungsgemäßen U-förmigen Stützelement 4 und einem Stoffstrang 20 entsteht. Weiterhin zeigt die Ausführungsform des offenbarungsgemäßen U-förmigen Stützelements 4 an den beiden Endabschnitten 8 und 10 Aufnahmeelemente 26 und 28. Diese sind in der gezeigten Variante als zweiteilige Durchführelemente ausgebildet, sodass ein Stoffstrang 20 durch diese Aufnahmeelemente 26 und 28 geführt werden kann (nicht gezeigt). Die Durchführelemente sind hier als schlitzförmige Materialaussparungen ausgebildet, wobei diese hier parallel zueinander verlaufen.

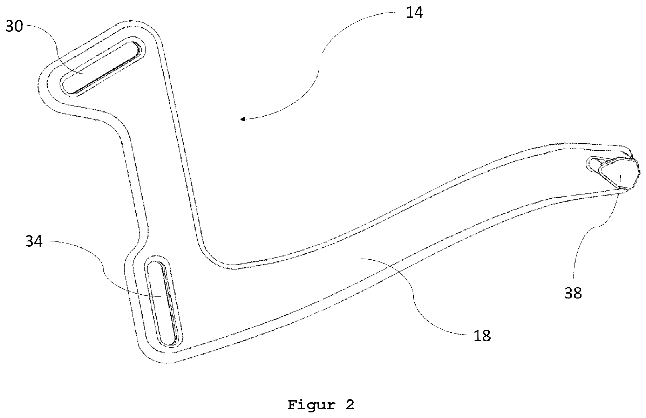

Figur 2 zeigt ein offenbarungsgemäßes erstes Übergangselement 14 aus einem steifen Material 18, welches hier als linksseitiges Übergangselement 14 ausgebildet ist, sich also beim Tragen auf der linken Gesichtshälfte des Patienten befindet. Das gezeigte Übergangselement 14 ist hier im Wesentlichen L-förmig ausgebildet mit einer längeren Seite und einer kürzeren Seite, wobei das Verhältnis der Seitenlängen hier 1,5 : 1 beträgt.

In der gezeigten Variante umfasst das Übergangselement 14 an den beiden Enden der kurzen Seite der L-Form ein erstes und ein zweites Aufnahmeelement 30 und 34, durch welche ein Stoffstrang durchgeführt werden kann (hier nicht gezeigt). In der hier gezeigten Variante des offenbarungsgemäßen Übergangselements 14 sind die Aufnahmeelemente 30 und 34 als einteilige schlitzförmige Materialaussparungen ausgebildet, wobei die beiden Aufnahmeelemente 30 und 34 hier in einem Winkel kleiner als 90° zueinander versetzt stehen. In der gezeigten Ausführungsform des ersten Übergangselements 14 weist dieses am Ende der langen Seite der L-Form ein Anschlusselement 38 zur Verbindung mit einem Patienteninterface 2 auf. In der hier gezeigten Variante ist dieses als Einrastelement ausgebildet, wobei der Gegenpart am Patienteninterface 2 eine zugehörige Aufnahmevorrichtung aufweist, das System also ein Klicksystem bildet (hier nicht gezeigt).

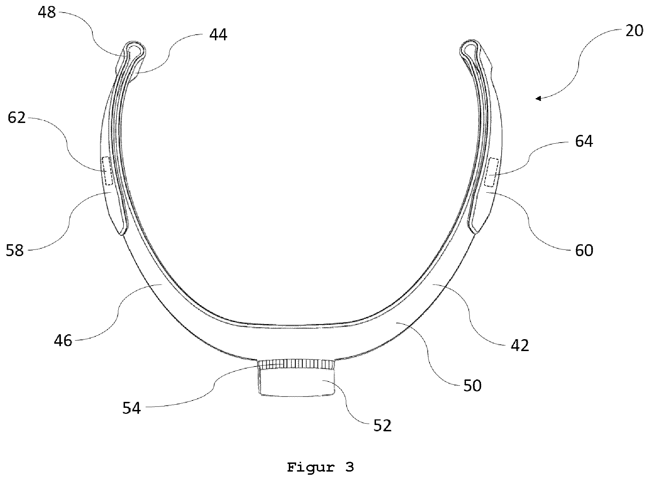

Figur 3 zeigt einen offenbarungsgemäßen
ersten Stoffstrang 20. In der gezeigten Ausführungsform ist dieser aus einem laminierten Verbundmaterial 42 hergestellt und umfasst eine patientenzugewandte Innenlage 44, eine Außenlage 46 und eine Zwischenlage 48, wobei die Innenlage 44 und die Außenlage 46 aus einem synthetischen Textilgewebe 50 bestehen. Die hier dargestellte Variante des ersten Stoffstrangs 20 umfasst mittig des Stoffstrangs 20 eine Lasche 52, welche über ein Verbindungselement 54 mit dem Stoffstrang 20 verbunden ist, wobei das Verbindungselement 54 hier als Naht ausgebildet ist. Um einen möglichst flachen Übergang zu generieren, wurden hier der Stoffstrang 20 und die Lasche 52 derart vernäht, dass diese nur aneinandergrenzen, jedoch nicht überlappen. Die gezeigte Lasche 52 ist ausgebildet, um lösbar in das Aufnahmeelement 24 des U-förmigen Stützelements 4 gemäß Figur 1 eingeführt zu werden. Der gezeigte Stoffstrang 20 weist ein erstes und ein zweites Strangende 58 und 60 auf. In der gezeigten Variante weisen die beiden Strangenden 58 und 60 jeweils ein Fixierungselement 62 und 64 auf, welches hier auf der patientenabgewandten Außenlage 46 angebracht ist in Form eines Hakenbands. Dabei ist dieses in der gezeigten Variante so ausgebildet, das es mit der kompletten Außenlage 46 nach dem Umschlagen verbunden werden kann. Die Strangenden 58 und 60 sind geeignet, um durch die Aufnahmeelemente 26 und 28 des U-förmigen Stützelements 4 der Figur 1 geführt zu werden.

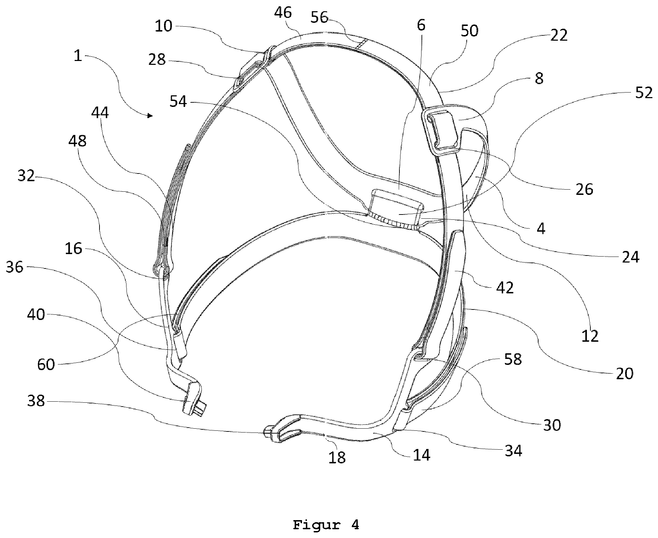

Figur 4 zeigt eine offenbarungsgemäße Befestigungsvorrichtung 1 für ein Patienteninterface 2 (hier nicht gezeigt). In der hier gezeigten Ausführungsform ist das U-förmige Stützelement 4 gemäß Figur 1 mit einem ersten Übergangselement 14 gemäß Figur 2 und einem weiteren zweiten Übergangselement 16, welches hier das rechtsseitige Übergangselement bildet, also auf der rechten Gesichtshälfte eines Patienten liegt, über einen ersten Stoffstrang 20 gemäß Figur 3 und einem weiteren Stoffstrang 22 verbunden. In der hier gezeigten Variante ist der erste Stoffstrang 20 über die Lasche 52 in das Aufnahmeelement 24 am Scheitelpunkt 6 des U-förmigen Stützelements 4 eingeführt und weiterhin über die Strangenden 58 und 60 in die zweiten Aufnahmeelemente 34 und 36 der Übergangselemente 14 und 16. Der zweite Stoffstrang 22 ist über seine Strangenden 58 und 60 zunächst durch die Aufnahmeelemente 26 und 28, hier als Durchführelemente ausgebildet, geführt und weiterhin durch die ersten Aufnahmeelemente 30 und 32 der Übergangselemente 14 und 16. Die Durchführung durch die Aufnahmeelemente 26 und 28, welche hier als zweiteilige Materialaussparungen ausgebildet sind, erfolgt hierbei in der Art, dass eine Erhöhung durch teilweises Aufliegen des zweiten Stoffstrangs 22 auf dem U-förmigen Stützelement 4 auf der patientenabgewandten Seite und nicht auf der patientenzugewandten Innenseite entsteht. Dabei ist der zweite Stoffstrang 22 so zwischen dem ersten und zweiten Endabschnitt 8 und 10 des U-förmigen Stützelements 4 platziert, dass das Kennzeichenelement 56 mittig zu diesen Endabschnitten 8 und 10 positioniert liegt. In bestimmungsgemäßem Gebrauch der offenbarungsgemäßen Befestigungsvorrichtung 1 dient das Kennzeichenelement 56 dem Patienten dazu, die Befestigungsvorrichtung 1 mittig auf dem Hinterkopf platzieren zu können. In der gezeigten Variante der offenbarungsgemäßen Befestigungsvorrichtung 1 spannen die Schenkel in Form der längeren und kürzeren Seite der L-förmigen Übergangselemente 14 und 16 jeweils eine Fläche xy auf. Die L-förmigen Übergangselemente 14 und 16 sind in der gezeigten Ausführungsform nicht flach in der Fläche xy ausgebildet, sondern bilden eine Erhöhung in die z-Ebene aus. Dadurch sind die beiden L-förmigen Übergangselemente 14 und 16 aufeinander zugehend ausgerichtet, um ein Patienteninterface 2 (nicht gezeigt) aufnehmen zu können. In der gezeigten Variante weisen die L-förmigen Übergangselemente 14 und 16 ferner bogenförmige Griffstrukturen auf der patientenabgewandten Seite auf, welche dem Patienten das Greifen der Übergangselemente 14 und 16 beim Befestigen mit einem Patienteninterface 2 (nicht gezeigt) erleichtern soll.

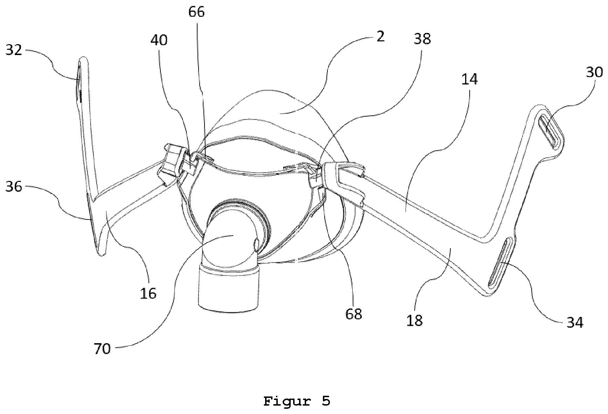

Figur 5 zeigt die bereits in Figur 4 gezeigten Übergangselemente 14 und 16, wobei diese hier über ihre Anschlusselemente 38 und 40 mit einem Patienteninterface 2 verbunden sind. In der hier gezeigten Variante umfasst das Patienteninterface 2 zwei Eingliederungselemente 66 und 68, welche die Anschlusselemente 38 und 40 der Übergangselemente 14 und 16 aufnehmen. Dies ist in der gezeigten Ausführungsform über einen Einrastmechanismus geregelt. Das gezeigte Patienteninterface 2 umfasst weiterhin ein Anbindungselement 70 zur Aufnahme eines Patientenschlauchs 102 (hier nicht gezeigt). In der hier gezeigten Ausführungsform umfasst das Anbindungselement 70 ein Kugelgelenk und das Patienteninterface 2 eine kugelförmige Materialaussparung zur Aufnahme dieses Kugelgelenks (hier nicht gezeigt).

### Liste der Bezugszeichen

- 1: Befestigungsvorrichtung
- 2: Patienteninterface
- 4: U-förmiges Stützelement
- 6: Scheitelpunkt des U-förmigen Stützelements
- 8: erster Endabschnitt des U-förmigen Stützelements
- 10: zweiter Endabschnitt des U-förmigen Stützelements
- 12: steifes und/oder habsteifes Material des U-förmigen Stützelements
- 14: erstes Übergangselement
- 16: zweites Übergangselement
- 18: steifes und/oder habsteifes Material der Übergangselemente
- 20: erster Stoffstrang
- 22: zweiter Stoffstrang
- 24: Aufnahmeelement am Scheitelpunkt
- 26: Aufnahmeelement am ersten Endabschnitt
- 28: Aufnahmeelement am zweiten Endabschnitt
- 30: erstes Aufnahmeelement des ersten Übergangselements
- 32: erstes Aufnahmeelement des zweiten Übergangselements
- 34: zweites Aufnahmeelement des ersten Übergangselements
- 36: zweites Aufnahmeelement des zweiten Übergangselements
- 38: Anschlusselement des ersten Übergangselements
- 40: Anschlusselement des zweiten Übergangselements
- 42: laminiertes Verbundmaterial
- 44: patientenzugewandte Innenlage
- 46: patientenabgewandte Außenlage
- 48: Zwischenlage
- 50: synthetisches Textilgewebe
- 52: Lasche
- 54: Verbindungselement
- 56: Kennzeichenelement
- 58: erstes Strangende
- 60: zweites Strangende
- 62: Fixierungselement am ersten Strangende
- 64: Fixierungselement am zweiten Strangende
- 66: erstes Eingliederungselement
- 68: zweites Eingliederungselement
- 70: Anbindungselement
- 102: Patientenschlauch

## Patentansprüche

1. Befestigungsvorrichtung (1) für ein Patienteninterface (2) umfassend ein im Wesentlichen U-förmiges Stützelement (4) mit einem Scheitelpunkt (6), sowie einem ersten (8) und einem zweiten (10) Endabschnitt, ausgebildet und eingerichtet im bestimmungsgemäßen Gebrauch den Hinterkopf eines Patienten zu umrahmen, wobei der Scheitelpunkt (6) ausgebildet und eingerichtet ist, bei einer Anwendung am Patienten mittig an dessen Hinterhauptbein anzuliegen und der erste (8) und der zweite Endabschnitt (10) ausgebildet und eingerichtet sind, bei einer Anwendung am Patienten jeweils flach seitlich an dessen Kopf über den Ohren positioniert zu sein,
wobei das U-förmige Stützelement (4) ein steifes und/oder halbsteifes Material (12) umfasst, wobei die Befestigungsvorrichtung ferner ein erstes (14) und ein zweites (16) Übergangselement umfasst, und ausgebildet und eingerichtet ist, das U-förmige Stützelement (4) mit dem Patienteninterface (2) zu verbinden, wobei das erste (14) und das zweite (16) Übergangselement, ein steifes und/oder halbsteifes Material (18) umfassen und im Wesentlichen L-förmig ausgebildet sind,
wobei die Befestigungsvorrichtung ferner einen ersten (20) und einen zweiten Stoffstrang (22) umfasst, welche ausgebildet und eingerichtet sind, das U-förmige Stützelement (4) mit dem ersten (14) und zweiten (16) Übergangselement, zu verbinden,
wobei das U-förmige Stützelement (4) ein Aufnahmeelement (24, Figur 1) am Scheitelpunkt zur Aufnahme des ersten Stoffstrangs (20), sowie ein Aufnahmeelement (26, 28), an den beiden Endabschnitten (8, 10), zur Aufnahme des zweiten Stoffstrangs (22), umfasst,
wobei das erste (14) und das zweite (16) Übergangselement ein erstes Aufnahmeelement (30, 32) zur Aufnahme des zweiten Stoffstrangs (22) und ein zweites Aufnahmeelement (34, 36) zur Aufnahme des ersten Stoffstrangs (20) umfassen,
wobei das erste (14) Übergangselement und das zweite (16) Übergangselement aus einem Material (18) ausgebildet sind, das eine höhere Steifigkeit als das U-förmige Stützelement (4) aufweist.

2. Befestigungsvorrichtung (1) nach Anspruch 1, wobei das erste (14) und/oder das zweite (16) Übergangselement ferner jeweils ein Anschlusselement (38, 40) zur Verbindung mit einem Patienteninterface (2) umfassen.

3. Befestigungsvorrichtung (1) nach Anspruch 1,
wobei der erste (20) und/oder der zweite (22) Stoffstrang ein laminiertes Verbundmaterial (42) umfassen mit einer dem patientenzugewandten Innenlage (44), einer Außenlage (46) und mindestens einer Zwischenlage (48),
wobei die Innen- (44) und/oder Außenlage (46) ein synthetisches Textilgewebe (50) und/oder cellulosische Chemiefasern umfassen,
wobei das synthetische Textilgewebe (50) mittels Spinnfärbung (dope dyed) eingefärbt ist.

4. Befestigungsvorrichtung (1) nach Anspruch 1 oder 3, wobei der erste (20) und/oder der zweite (22) Stoffstrang ferner eine Lasche (52) umfassen, ausgebildet und eingerichtet in ein Aufnahmeelement (24, 26, 28, 30, 32, 34, 36) eingeführt zu werden, und/oder wobei der erste Stoffstrang (20) und/oder der zweite (22) Stoffstrang und die Lasche (52) über ein Verbindungselement (54) miteinander verbunden sind.

5. Befestigungsvorrichtung (1) nach einem der Ansprüche 1 oder 3, wobei der erste (20) und/oder der zweite (22) Stoffstrang ein Kennzeichenelement (56) umfassen, wobei das Kennzeichenelement (56) mittig zu einem ersten (58) und einem zweiten Strangende (60) verläuft, wobei das Kennzeichenelement (56) ausgebildet und eingerichtet ist, beim Durchführen des ersten (20) und/oder zweiten (22) Stoffstrangs durch die Aufnahmeelemente (24, 26, 28) des U-förmigen Stützelements (4) mittig zu den beiden Endabschnitten (8, 10) zu sitzen.

6. Befestigungsvorrichtung (1) nach einem der Ansprüche 4 oder 5, ferner umfassend jeweils ein Fixierungselement (62, 64) an dem ersten (58) und an dem zweiten (60) Strangende des ersten (20) und/oder zweiten (22) Stoffstrangs, ausgebildet und eingerichtet, die Übergangselemente (14, 16) zur Verbindung mit einem Patienteninterface (2) aufzunehmen.

7. Befestigungsvorrichtung (1) nach Anspruch 6, wobei die Fixierungselemente (62, 64) ausgebildet und eingerichtet sind, die Übergangselemente (14, 16) so aufzunehmen, dass gleichzeitig eine Größenanpassung an die Kopfform des Patienten ermöglicht wird.

8. System zur Beatmung und/oder Atemunterstützung umfassend ein Patienteninterface (2), eine Befestigungsvorrichtung (1) und einen Patientenschlauch (102), wobei das Patienteninterface (2) über den Patientenschlauch (102) mit einem Beatmungsgerät und/oder das Atemtherapiegerät gasleitend in Verbindung steht, wobei die Befestigungsvorrichtung (1) nach mindestens einem der vorangehenden Ansprüche 1 bis 7 ausgebildet ist.

## Claims

1. A fastening device (1) for a patient interface (2),
comprising a substantially U-shaped support element (4) having an apex (6), and a first (8) and a second (10) end portion, designed and configured, in the intended use, to frame the back of a patient's head, wherein the apex (6) is designed and configured, when used on the patient, to bear centrally against the patient's occipital bone, and wherein the first (8) and the second (10) end portions are designed and configured, when used on the patient, to be positioned, in each case, flat against the side of the patient's head above the ears,
wherein the U-shaped support element (4) comprises a rigid and/or semi-rigid material (12), wherein the fastening device further comprises a first (14) and a second (16) transition element, and is designed and configured to connect the U-shaped support element (4) to the patient interface (2),
wherein the first (14) and the second (16) transition elements comprise rigid and/or semi-rigid material (18) and are designed to be substantially L-shaped,
wherein the fastening device further comprises a first (20) and a second fabric strand (22), which are designed and configured to connect the U-shaped support element (4) to the first (14) and second (16) transition element,
wherein the U-shaped support element (4) comprises a receiving element (24, Fig. 1) at the apex for receiving the first fabric strand (20), and a receiving element (26, 28) at the two end portions (8, 10) for receiving the second fabric strand (22),
wherein the first (14) and the second (16) transition element each comprise a first receiving element (30, 32) for receiving the second fabric strand (22) and a second receiving element (34, 36) for receiving the first fabric strand (20),
wherein the first (14) transition element and the second (16) transition element are formed from a material (18) that has a higher rigidity than the U-shaped support element (4).

2. A fastening device (1) according to claim 1,
wherein the first (14) and/or the second (16) transition element further comprises, in each case, a connection element (38, 40) for connecting to a patient interface (2).

3. The fastening device (1) according to claim 1,
wherein the first (20) and/or the second (22) fabric strand comprises a laminated composite material (42) having a patient-facing inner layer (44), an outer layer (46), and at least one intermediate layer (48),
wherein the inner (44) and/or outer layer (46) comprise a synthetic textile fabric (50) and/or cellulosic man-made fibers, wherein the synthetic textile fabric (50) is dope-dyed.

4. The fastening device (1) according to claim 1 or 3,
wherein the first (20) and/or the second (22) fabric strand further comprises a tab (52) that is designed and configured to be inserted into a receiving element (24, 26, 28, 30, 32, 34, 36), and/or wherein the first fabric strand (20) and/or the second (22) fabric strand and the tab (52) are connected to one another via a connecting element (54).

5. The fastening device (1) according to one of claims 1 to 3,
wherein the first (20) and/or the second (22) fabric strand comprises a marking element (56), wherein the marking element (56) extends centrally between a first (58) strand end and a second strand end (60), wherein the marking element (56) is designed and configured to be seated centrally with respect to the two end portions (8, 10) when the first (20) and/or second (22) fabric strand is passed through the receiving elements (24, 26, 28) of the U-shaped support element (4).

6. The fastening device (1) according to one of claims 4 to 5, further comprising, in each case, a securing element (62, 64) at the first (58) and at the second (60) strand end of the first (20) and/or second (22) fabric strand that is designed and configured to receive the transition elements (14, 16) for connecting to a patient interface (2).

7. The fastening device (1) according to claim 6,
wherein the securing elements (62, 64) are designed and configured to receive the transition elements (14, 16) such that it is possible to simultaneously adjust the size to the shape of the patient's head.

8. A system for ventilation and/or respiratory support, comprising a patient interface (2), a fastening device (1), and a patient tube (102), wherein the patient interface (2) is connected in a gas-conducting manner to a ventilator and/or the respiratory therapy device via the patient tube (102), wherein the fastening device (1) is designed according to at least one of the preceding claims 1 to 7.

## Revendications

1. Dispositif de fixation (1) pour une interface patient (2) comportant un élément de support essentiellement en forme de U (4) présentant un sommet (6) ainsi qu'une première (8) et une deuxième (10) sections d'extrémité, conçu et configuré pour entourer l'arrière de la tête d'un patient lors d'une utilisation conforme, dans lequel le sommet (6) est conçu et configuré pour venir s'appliquer de manière centrée sur l'os occipital du patient lors d'une utilisation sur celui-ci, et la première (8) et la deuxième section d'extrémité (10) sont conçues et configurées pour être positionnées respectivement au-dessus des oreilles, à plat latéralement sur la tête du patient, lors d'une utilisation sur celui-ci,
dans lequel l'élément de support en forme de U (4) comporte un matériau rigide et/ou semi-rigide (12), dans lequel le dispositif de fixation comporte en outre un premier (14) et un deuxième (16) éléments de transition, tout en étant conçu et configuré pour relier l'élément de support en forme de U (4) à l'interface patient (2), dans lequel le premier (14) et le deuxième (16) éléments de transition comportent un matériau rigide et/ou semi-rigide (18) et sont conçus essentiellement en forme de L,
dans lequel le dispositif de fixation comporte en outre une première (20) et une deuxième bande de tissu (22), lesquelles sont conçues et configurées pour relier l'élément de support en forme de U (4) au premier (14) et au deuxième (16) éléments de transition,
dans lequel l'élément de support en forme de U (4) comporte un élément de réception (24, Figure 1) au niveau du sommet, destiné à recevoir la première bande de tissu (20), ainsi qu'un élément de réception (26, 28) au niveau des deux sections d'extrémité (8, 10), destiné à recevoir la deuxième bande de tissu (22),
dans lequel le premier (14) et le deuxième (16) éléments de transition comportent un premier élément de réception (30, 32) destiné à recevoir la deuxième bande de tissu (22) ainsi qu'un deuxième élément de réception (34, 36) destiné à recevoir la première bande de tissu (20),
dans lequel le premier (14) élément de transition et le deuxième (16) élément de transition sont constitués d'un matériau (18) présentant une rigidité supérieure à celle de l'élément de support en forme de U (4).

2. Dispositif de fixation (1) selon la revendication 1,
dans lequel le premier (14) et/ou le deuxième (16) éléments de transition comportent en outre respectivement un élément de raccordement (38, 40) destiné à être relié à une interface patient (2).

3. Dispositif de fixation (1) selon la revendication 1,
dans lequel la première (20) et/ou la deuxième (22) bandes de tissu comportent un matériau composite stratifié (42) comprenant une couche intérieure (44) tournée vers le patient, une couche extérieure (46) et au moins une couche intermédiaire (48),
dans lequel la couche intérieure (44) et/ou la couche extérieure (46) comportent un tissu textile synthétique (50) et/ou des fibres chimiques cellulosiques, le tissu textile synthétique (50) étant teint dans la masse (dope dyed).

4. Dispositif de fixation (1) selon la revendication 1 ou 3,
dans lequel la première (20) et/ou la deuxième (22) bande de tissu comportent en outre une languette (52) conçue et configurée pour être introduite dans un élément de réception (24, 26, 28, 30, 32, 34, 36), et/ou dans lequel la première bande de tissu (20) et/ou la deuxième (22) bande de tissu et la languette (52) sont reliées entre elles par un élément de liaison (54).

5. Dispositif de fixation (1) selon l'une quelconque des revendications 1 ou 3,
dans lequel la première (20) et/ou la deuxième (22) bande de tissu comportent un élément de marquage (56), l'élément de marquage (56) s'étendant au centre par rapport à une première (58) et une deuxième extrémité de bande (60), l'élément de marquage (56) étant conçu et configuré pour être placé au centre par rapport aux deux sections d'extrémité (8, 10) lors du passage de la première (20) et/ou de la deuxième (22) bandes de tissu à travers les éléments de réception (24, 26, 28) de l'élément de support en forme de U (4).

6. Dispositif de fixation (1) selon l'une des revendications 4 ou 5,
comportant en outre respectivement un élément de fixation (62, 64) à la première (58) et à la deuxième (60) extrémité de bande de la première (20) et/ou de la deuxième (22) bandes de tissu, conçu et configuré pour recevoir les éléments de transition (14, 16) en vue du raccordement à une interface patient (2).

7. Dispositif de fixation (1) selon la revendication 6,
dans lequel les éléments de fixation (62, 64) sont conçus et configurés pour recevoir les éléments de transition (14, 16) de telle façon qu'il est possible d'adapter simultanément la taille à la forme de la tête du patient.

8. Système de ventilation et/ou d'assistance respiratoire comportant une interface patient (2), un dispositif de fixation (1) et un tube de patient (102), dans lequel l'interface patient (2) est en communication fluidique avec un ventilateur et/ou un appareil de thérapie respiratoire par le biais du tube de patient (102), dans lequel le dispositif de fixation (1) est conçu selon l'une au moins des revendications précédentes 1 à 7.
